Europäisches Patentamt

European Patent Office

Office européen des brevets

(19)

(11) Publication number: 0 204 045
A2

## (12) EUROPEAN PATENT APPLICATION

(21) Application number: 85304745.4

(22) Date of filing: 03.07.85

(51) Int. Cl.⁴: G 01 N 33/48
C 12 Q 1/56, G 01 N 33/96
A 61 J 3/10, A 61 K 9/20

(30) Priority: 05.06.85 US 741418

(43) Date of publication of application:
10.12.86 Bulletin 86/50

(84) Designated Contracting States:
AT BE CH DE FR GB IT LI LU NL SE

(71) Applicant: Bio/Data Corporation
3615 Davisville Road
Hatboro Pennsylvania 19040(US)

(72) Inventor: Lawson, Daniel E.
217 Fulling Mill Lane
Ambler Pennsylvania 19002(US)

(72) Inventor: Messa, Eugene J.
880 Pebble Hill Road
Doylestown Pennsylvania 18901(US)

(72) Inventor: Sokol, Michael
7617 Lycoming Avenue
Melrose Park Pennsylvania 19126(US)

(72) Inventor: Werner, Belinda S.
291 Beth Ann Drive
Harleysville Pennsylvania 19438(US)

(74) Representative: Oliver, Roy Edward et al,
POLLAK MERCER & TENCH High Holborn House 52-54
High Holborn
London WC1V 6RY(GB)

(54) Tableted blood plasma microconcentrated coagulation reagents and method of making same.

(57) A blood plasma coagulation reagent is prepared in micro-concentrated tablet form by dry blending a binder and lubricant with a lyophilized active ingredient or ingredients and buffer, or dry blending a lubricant with a lyophilized active ingredient or ingredients and binder solution. In another related method a solution of the lyophilized active ingredient and binder is sprayed onto granules of lyophilized buffer. The dry blended mix is compacted, granulated and tableted. Two or more dry blended batches may be combined prior to compaction to increase the size of the final manufacturing batch.

EP 0 204 045 A2

- 1 -

## TABLETED BLOOD PLASMA MICROCONCENTRATED COAGULATION REAGENTS AND METHOD OF MAKING SAME

### Background Of The Invention

As is evident from Monte et al U.S. Patent No. 3,997,470, it has been known to tablet reagents for assaying biological specimens. However, the inventors do not know of any prior art tablets, particularily microconcentrated tablets, which contain blood plasma coagulation reagents.

Further, the method of preparation disclosed in the Monte et al patent is a wet granulation method which requires a specified surfactant and a drying cycle to drive off the solvent in which the ingredients of the tablet are dissolved. Therefore, the Monte et al process requires an additional time consuming step which increases manufacturing costs and may require the addition of heat.

Still further, the batch or lot size is limited to the amount of formulation which may be dried in a single lyophilizer cycle. Typical industry practice limits a batch or lot size to the number of vials which can be contained and stoppered within the capacity of the lyophilizer in use.

Prior methods of preparing blood plasma coagulation reagents have typically involved compounding a formulation including a filler, apportioning aliquots of the formulation into vials, lyophilizing the aliquots in the vials and sealing the lyophilized aliquots in the vials. In an attempt to maintain uniformity within the batch, the amount of time taken to meter out the aliquots, fill the vials, and seal the vials is minimized. However, it is not unusual to loose ten, fifteen percent or more of the batch due to nonuniformity between vials as a result of the nonuniformity within the lyophilizer during the lyophilization process.

## Summary Of The Invention

One object of the present invention is to provide a method of tableting blood plasma coagulation reagents in which the batch size is not limited to the amount of formulation which may be lyophilized.

It is a further object to provide a method of preparing microconcentrated blood plasma coagulation reagents in tablet form which does not require the use of a solvent or heat which may be detrimental to the coagulation reagents.

It is still a further object to provide a method of tableting microconcentrated blood plasma coagulation reagents which is simple and economical.

Yet another object is to provide a method of tableting blood plasma coagulation reagents in microconcentrated tablet form by coating one ingredient which is in granular form with a second ingredient.

It is another object of the present invention to provide lyophilized blood plasma coagulation reagents in microconcentrated tablet form which are inexpensive and which have a long shelf life and are convenient to use.

It is still another object to prepare blood plasma coagulation reagents in microconcentrated tablet form without loss of function and/or performance characteristics as compared to lyophilized vials of the same materials.

Other objects and advantages will appear hereinafter.

The above objects are accomplished by tableting lyophilized microconcentrated blood plasma coagulation reagents in a tableting process in which the active blood plasma coagulation reagents are lyophilized and mixed with tableting excipients or sprayed onto granules of a lyophilized buffer. Two or more lots of the bulk lyophilized reagents are dry blended to form a larger manufacturing
batch prior to compacting, granulation and tableting.

The present invention is directed to reagents for
use in blood plasma coagulation tests performed either for
medical diagnostic purposes or for monitoring of certain
anticoagulant drug therapies. Specifically, the reagents
include thrombin, thromboplastin, cephalin and four control
plasmas. The four control plasmas include a normal plasma,
two abnormal plasmas and a heparinized plasma.

## Detailed Description

The process will first be described with respect
to the formulation and production of bovine thrombin tablets.
However, it is to be understood that the specific examples
are illustrative only and the scope of the invention is
to be determined by the appended claims. Further, other
types of thrombin such as human thrombin may be substituted
for the bovine thrombin.

The described process yields approximately 1,000
tablets, each tablet having about 8 to 10 U.S.P. (United
States Pharmacopeia) units of bovine thrombin. It is obvious
that the number of units per tablet and the size of the
batch may be varied without departing from the essence of
the invention. The working concentration of 8 to 10 U.S.P.
units of thrombin is chosen since this is the amount used
in thrombin time tests in many laboratories and which
results in a thrombin time of about 15 seconds to about 18
seconds on normal fresh plasma. Other concentrations of
thrombin with different thrombin time results may be used
without departing from the essence of the invention.

For convenience the bovine thrombin may be purchased
in a 10,000 U.S.P. unit ampoule. However, the bovine
thrombin can be purchased in other concentrations such as
100,000 U.S.P. units per ampoule and diluted. Alternatively,
bovine whole blood may be purchased and the thrombin extracted
from the whole blood. The bovine thrombin has a pH range
of 6.5 to 8.5.

The active reagent, bovine thrombin, is mixed with a buffer, saline, an antimicrobial preservative and bovine serum albumin to form a generic reagent which is lyophilized.

A preferred buffer is HEPES (N-2-hydroxyethyl piperazine-N'-2-ethane sulfonic acid) having a pH of about 8.0. It is preferred to use a molar concentration of 0.025M. However, the concentration can vary from between about 0.01M, where the buffer looses its effect, to about 0.6M, where the buffer begins to interfere with the thrombin time test. The pH of the HEPES should be about 6.2 to about 8.5. Other buffers which may be used include imidazole, tris [tris(hydroxymethyl)aminomethane], glycine and other buffers in the 8.0 range.

The saline is a 0.85% sodium chloride solution. While the concentration of the solution may be varied, it is preferred to use 0.85%.

The preferred antimicrobial preservative is thimerosal (sodium ethylmercurithiosalicyate). However, other preservatives such a sodium azide may be used. It is preferred to use a 0.02% solution of thimerosal. However, the concentration may vary between about 0.01% and about 0.3%. Below 0.01% the thimerosal loses its effect and above 0.3% there is no additional effect.

The bovine serum albumin (BSA) reduces the tendency of thrombin to adhere to nonsiliconized glass during the thrombin test and it also aids in stabilizing the reagent. The concentration of the BSA may vary between about 0.005% and about 3.0%. However, it is preferred to use a concentration of 0.01%.

A solution of the buffer, saline, preservative and BSA is formed by preferably mixing one liter of the HEPES, one liter of the saline, 200 milligrams of the thimerosal and 1 gram of the BSA. The amount of the buffer and saline may vary between about 500 milliliters and about 2 liters. The amount of the thimerosal may be varied between about 100 milligrams and about 400 milligrams and the BSA may be varied between about 0.5 grams and about 3.0 grams.

This solution is adjusted to a pH of about 8.0 with the addition of 5N sodium hydroxide. Then 10,000 units of the bovine thrombin is added to the solution.

The solution is then lyophilized in bulk. The solution is dried to a maximum moisture content of no more than about 1.0% to about 3.0%. The lyophilized reagent is dry blended with a binder and lubricant to form a blend which is to be compacted, granulated and tableted. However, the lubricant may be added after the lyophilized reagent and binder are granulated.

The preferred binder is Di-Pac (a registered trademark of Amstar Corporation). Di-Pac is made up of about 97.0% sucrose and about 3.0% malto-dextrin. However, other binders such as sucrose, maltose, mannitol and lactose may be used. In addition, other sugars, acacia, gelatin, povidone, methylcellulose, carboxymethyl cellulose and hydrolized starch pastes may be used with the bovine thrombin and the other coagulation reagents.

The preferred lubricant is L-leucine. Other lubricants which may be used include polyethylene glycol (PEG), magnesium stearate and talc, as well as other metallic stearates, stearic acid and hydrogenated vegetable oils.

Preferably, 20.0% by weight of the lyophilized reagent, 70.0% of the binder and 10.0% of the lubricant are dry blended. However, the amounts of these components may vary from about 7.0% to about 38.0% of the lyophilized reagent, about 20.0% to about 90.0% of the binder and about 3.0% to about 12.0% of the lubricant. More preferably, the lyophilized reagent is in an amount from about 9.0% to about 35.0%, the binder is in an amount from about 60.0% to about 80.0% and the lubricant is in an amount of about 5.0% to about 11.0%.

The dry blend is tested by running a number of 25 milligram samples of the dry blend in a thrombin time test. If the tests yield a result of less than 15 seconds, additional Di-Pac may be added to dilute the blend and lengthen the time for the test. If the results of the tests are greater than 18 seconds, additional lyophilized reagent which was held back is added to the dry blend to decrease the test time. The test times may also be corrected by adjusting the sample size and therefore the amount of dry blend in each tablet. However, it is desired to maintain the tablet size of between about 20 milligrams and about 35 milligrams. Another thrombin time test may be run to verify that the adjustment has brought the time within the desired range.

After two or more of the dry blends have been prepared and tested, they are combined to form a final manufacturing batch. The manufacturing batch is obviously larger than the amount of formulation which may be lyophilized in a batch lyophilizer. The advantage of such a larger manufacturing batch and the tablets obtained from that batch is that the reagent may be used by a larger number of laboratories thereby facilitating interlaboratory quality control comparisons.

The dry blended batches have a low bulk density and are not readily flowable. Therefore, the dry blended manufacturing batch is compacted to increase bulk density. The resultant compaction is granulated to make it flowable and minimize fines. The granules are screened to adjust the size of the granules used in the tableting process. The oversized granules may be further ground and the fines may be recompacted and regranulated.

Prior to tableting the granulated material, a sample is taken and another thrombin time test run to verify the amount of material by weight which should be used per tablet. If necessary, the size of the tablet is adjusted to yield a result of between 15 and 18 seconds as previously described.

The activity of the bovine thrombin solution may be adjusted prior to lyophilization by testing the activity of the solution in a thrombin time test. However, it has been found that the activity may be adequately adjusted after lyophilization as previously described.

While the preferred process has been described, elimination of certain of the ingredients such as the saline, preservative and BSA will produce a satisfactory microconcentrated tablet. However, the absence of such components may affect long term unreconstituted stability and short term reconstituted stability.

The next reagent to be discussed is thromboplastin. Thromboplastin is a phospholipid extract from such organs as rabbit brain, human brain, monkey brain, rabbit lung bovine brain, and ox brain. The thromboplastin may be extracted by the Quick Method as known in the art. A solution of the thromboplastin extract is formed by dissolving approximately 40.0 grams of the phospholipid source material in one liter of saline. Preferably, the saline solution is 0.5% sodium chloride. The concentration of the saline may be from about 0.25% to about 0.85%. The activity of the phospholipid extract is determined by running a prothrombin time test (PT test) as is well known in the art. The results should be about 10 seconds to about 13 seconds with fresh normal citrated plasma.

The lyophilized reagent is formed by mixing the phospholipid extract with a buffer, preservative and activating agents. The preferred buffer is 0.05M imidazole having a pH of about 7.35. Other buffers such as tris, barbitol and other buffers in the pH 7.0 range may be used.

The preferred preservative is thimerosal in the concentrations discussed with respect to the bovine thrombin. Other preservatives include neomycin sulfate, sodium azide and phenol or others known to inhibit microbial growth.

The activating agents include 0.0125M calcium chloride and maganese chloride, magnesium chloride or other divalent metal. The concentration of the calcium chloride may vary between about 0.01M and 0.02M. The preferred concentration of the divalent metal is 0.01M but may vary between about 0.005M and 0.02M.

One liter of each of the buffer, calcium chloride, divalent metal, and preservative are mixed and added to one liter of the extract. A sample of the solution is taken and a PT test run. The solution is adjusted to obtain a PT test result of between about 10 seconds to about 13 seconds with fresh normal citrated plasma.

The reagent solution is then lyophilized. The lyophilized reagent is then dry blended with a binder and lubricant.

The preferred binder is malto-dextrin such as AMAIZO LO-DEX 5 (a registered trademark of American Maize-Products Company). Other binders such as Di-Pac, mannitol or other binders discussed with regard to bovine thrombin may be used.

A preferred lubricant is PEG. However, other lubricants which may be used include magnesium stearate, L-leucine, gylcine and silica such as Cab-O-Sil (a registered trademark of Cabot Corporation).

Preferably, 3.0% by weight of the malto-dextrin and 3.0% by weight of the PEG are added to the lyophilized reagent. The amount of the malto-dextrin may be varied from about 1.0% to about 10.0% by weight and the amount of the PEG may be varied from about 1.0% to about 12.0% by weight.

If other binders are used, the amount added may need to be adjusted. It may be desirable to add a disintegrating agent such as a starch, cellulose or effervescent mixtures.

As with the bovine thrombin reagent, the dry blend batch is tested, adjusted as necessary to yield the proper test result time, and then is combined with other dry blend batches to form a final manufacturing batch which is compacted, granulated and tableted.

To prepare the Activated Partial Thromboplastin Time (APTT) reagent, cephalin may be extracted from rabbit brain by the method of Bell and Alton as is known in the art. Ten grams of the cephalin is blended with a liter of a 0.85% sodium chloride and 0.02% thimerosal solution. The cephalin and saline/preservative solution is homogenized for one minute. The concentrations of the saline and thimerosal may be varied and other preservatives used as discussed previously.

A 0.2M buffer (pH of about 7.5) containing 0.02% thimerosal and 0.85% saline is prepared. The preferred buffer is tris.

A 10.0% malto-dextrin solution containing fumed silica is prepared. The preferred malto-dextrin is AMAIZO LO-DEX 15 (a registered trademark of American Maize-Products Company). The concentration of the malto-dextrin can vary from between about 3.0% to about 20.0%. The concentration of the fumed silica should be between about 8.0 grams per liter and about 16.0 grams per liter. The preferred particle size of the fumed silica is a size no greater than 0.012 microns. Silica in other forms and particle sizes may likewise be used.

The cephalin solution is combined with the buffer solution in a ratio of about 50 milliliters of cephalin solution to 950 milliliters of the buffer solution. The mixture is stirred for one hour and a sample taken to run a PTT test. The result of the PTT test should be between about 60 seconds and about 85 seconds with fresh normal citrated plasma. The solution is adjusted as necessary to yield the desired test time.

The malto-dextrin solution is then added in the ratio of about 500 milliliters of the cephalin/buffer solution to 400 milliliters of the malto-dextrin solution. This mixture is stirred for one hour to activate the cephalin. An APTT test is then run to determine the activity of the reagent. While the above ratios are preferred, they may be varied. The solution is then lyophilized.

The lyophilized reagent is dry blended with a lubricant. Preferably, the lubricant is 10.0% by weight of L-leucine. The amount of the L-leucine may be varied from between about 1.0% to about 12.0% by weight. The dry blend batch is then combined with one or more other batches to form a final manufacturing batch. A sample of the manufacturing batch is tested to determine the amount to be used per tablet to obtain the proper test time as previously described.

After the dry blend is compacted and granulated, it is again tested to determine the proper amount to be used per tablet. The dry blend is then tableted.

Up to about 15.0% by weight of disintegrating agents may be added to the thrombin, thromboplastin and cephalin reagent formulations described earlier. The disintegrating agents which may be used include cellulose, starch and effervescent mixtures.

The normal plasma control coagulation reagent is prepared by centrifuging and removing the plasma from fresh normal whole blood drawn in Citrate-Phosphate-Dextrose-Adenine (CPDA-1). The ratio of the CPDA-1 to whole blood is 63 milliliters of CPDA-1 per 100 milliliters of whole blood, as known in the art. Ten milliliters of HEPES is added per 100 milliliters of centrifuged plasma. Preferably the HEPES is 0.02M (pH 7.4) containing 0.85% saline and 0.02% thimerosal.

Three tests are run to determine if the plasma is acceptable. The PT test, as previously described, should yield a time of about 10.0 seconds to about 13.0 seconds seconds. The APTT test, as previously described, should yield a time of about 28.0 seconds to about 38.0 seconds. The Thrombin Clotting Time (TCT) test, as previously described, should be yield a time of about 15.0 seconds to about 18.0 seconds.

The abnormal plasma controls are formed by adsorbing out certain clotting factors from normal plasma with aluminum hydroxide or barium sulfate and centrifuging the plasma as is known in the art. The test times for the level I abnormal plasma control should be about 16.0 seconds to about 20.0 seconds for the PT test and about 55.0 seconds to about 70.0 seconds for the APTT test. The level II abnormal plasma control should produce about 26.0 seconds to about 32.0 seconds for the PT test and about 80.0 seconds to about 105.0 seconds for the APTT test.

The heparin control plasma is formed by adding heparin to normal plasma. About 0.01 units to about 0.5 units of heparin is added per milliliter of plasma to form the heparin control plasma.

After the control plasmas have been prepared as discussed above, they are lyopholized. Each lyophilized plasma is dry blended with a binder, a lubricant and a disintegrating agent.

The preferred binder is a combination of Di-Pac and sucrose or trehalose. The relative ratios are about 10.0% Di-Pac and about 2.0% sucrose or trehalose by weight. The amount of the Di-Pac may be varied from about 5.0% to about 50.0%. The amount of the sucrose or trehalose may be varied from about 0.5% to about 20.0%. Preferably, the sucrose or trehalose is added to the pre-lyopholized plasma preparation. Other binders which may be used include other sugars such as sucrose.

The preferred lubricant is PEG in an amount of about 5.0% by weight. The amount of lubricant may be varied from between about 1.0% to about 15.0%. Other lubricants which may be used include L-leucine, magnesium stearate and glycine as well as other lubricants known in the art. If magnesium stearate is used, it is preferred to use less than 1.0% by weight.

Up to about 5.0% by weight of disintegrating agent may be added to the dry blend. The disintegrating agents which may be used include the celluloses and starches and effervescent mixtures.

As discussed previously, the dry blend tablet batch is then combined with one or more other dry blend batches to form a final manufacturing batch which is compacted and granulated. The granulated material is tested to determine the proper tablet size and is tableted.

The above described method may be modified by spraying a solution of lyophilized reagent and binder onto granules of the buffer. While it may be possible to modify each of the above described methods, only the production of the thromboplastin tablet will be described. One liter of the previously described imidazole buffer is lyophilized. If the divalent metal is to be included in the tablet, it is added to the buffer solution prior to lyophilization in the concentration and amounts previously set forth.

Approximately 40 grams of the thromboplastin phospholipid is dissolved in one liter of 0.85% saline solution. About 2.0% by weight of the binder, preferably trehalose, is added to the thromboplastin/saline solution. This solution is then sprayed on the granules of buffer and dried at about 10°C under a high vacuum of at least 30 microns for 24 hours.

The concentration of the trehalose may be varied between about 0.5% and about 10.0%. THe amount of the buffer may be varied between about 1.0% to about 10.0% by weight as previously discussed.

After the sprayed granules are dried, they are recompacted and regranulated. The lubricants set forth previously may be dry blended with the dry sprayed granules. However, it is preferred to add about 3.0% by weight of PEG, or other lubricant, after the regranulation and before tableting.

Alternatively, the method may be modified by spraying the buffer solution on granules of the lyophilized reagent and binder.

The tablets formed by the previously disclosed methods have a substantial long term unreconstituted stability and short term reconstituted stability. The tablets are convenient to use and handle. Storage is facilitated by their small physical size. They dissolve readily, are easily used and yield uniform results.

The preparation of the coagulation reagents in tablet form reduces the packaging costs. Previously, coagulation reagents were lyophilized in vials containing small amounts of the reagents. Not only has the expense of the vials been eliminated but the amount of each reagent manufacturing batch has been increased further reducing costs.

The present invention may be embodied in other specific forms without departing from the spirit or essential attributes thereof and, accordingly, reference should be made to the appended claims, rather than to the foregoing specification, as indicating the scope of the invention.

CLAIMS

1. A tableting process for preparing microconcentrated blood plasma coagulation reagents comprising preparing a solution containing an active coagulation reagent, lyophilizing the solution to form a lyophilized reagent, adding a buffer, adding a binder, adding a lubricant, compacting the active reagent, buffer and binder, granulating the compacted material, and tableting the granulated material.

2. The process according to claim 1 wherein the binder is dry blended with the lyophilized reagent prior to compacting.

3. The process according to claim 1 wherein the binder is added to the reagent solution prior to lyophilization.

4. The process according to claim 1 wherein a disintegrating agent selected from the group consisting of celulose, starch and effervescent mixtures is added in an amount no greater than about 15.0%.

5. The process according to claim 1 wherein at least two dry blend batches are dry blended to form a manufacturing batch prior to the compacting step.

6. The process according to claim 1 wherein the coagulation reagent is thrombin and wherein a saline solution, a preservative or bovine serum albumin is added to the active reagent prior to lyophilization.

7. The process according to claim 1 wherein the active reagent is thrombin and wherein the buffer is HEPES buffer having a pH of about 6.2 to about 8.5.

8. The process according to claim 1 wherein the active reagent is thrombin, and wherein the binder is selected from the group consisting of about 97.0% sucrose and about 3.0% malto-dextrin, sucrose, maltose, mannitol and lactose, and wherein the binder is dry blended with the lyophilized active reagent prior to compacting.

9. The process according to claim 1 wherein the active reagent is thrombin selected from the group consisting of bovine thrombin and human thrombin having

a pH of about 6.5 to about 8.5, wherein the buffer is HEPES having a pH of about 6.2 to about 8.5 and a concentration of about 0.01M to about 0.6M, wherein a saline solution of about 0.85%, thimerosal in a concentration of about 0.02% and bovine serum albumin in a concentration of about 0.01% is added to the active reagent prior to lyophilization, wherein the binder is about 97.0% sucrose and about 3.0% malto-dextrin and is dry blended with the lyophilized reagent in an amount of about 20.0% to about 90.0% by weight, and wherein the lubricant is L-leucine in an amount of about 1.0% to about 12.0% by weight.

10. The process according to claim 1 wherein the active reagent is a thromboplastin, and wherein calcium chloride is added to the active reagent prior to lyophilization.

11. The process according to claim 10 wherein a divalent metal is added prior to compacting.

12. The process according to claim 11 wherein the divalent ion is selected from the group consisting of magnesium and manganese.

13. The process according to claim 10 wherein the binder is added to the active reagent prior to lyophilization.

14. The process according to claim 10 wherein the binder is dry blended with the lyophilized active reagent.

15. The process according to claim 10 wherein the buffer is selected from the group consisting of imidazole, tris and barbitol.

16. The process according to claim 10 including the step of adding a preservative to the active reagent prior to lyophilization.

17. The process according to claim 16 wherein the preservative is selected from the group consisting of thimerosal, neomycin sulfate, sodium azide and phenol.

18. The process according to claim 10 wherein the binder is selected from the group consisting of malto-dextrin, mannitol, and a mixture of about 97.0% sucrose and about 3.0% malto-dextrin.

19. The process according to claim 10 wherein the lubricant is selected from the group consisting of polyethylene glycol, magnesium stearate, L-leucine, glycine and silica.

20. The process according to claim 10 wherein the buffer is imidazole, wherein a divalent metal selected from the group consisting of magnesium and manganese is added, wherein the preservative is thimerosal, wherein about 1.0% to about 10.0% of malto-dextrin binder is added to the active reagent prior lyophilization, and wherein about 1.0% to about 12.0% of polyethylene glycol lubricant is dry blended with the lyophilized active reagent.

21. The process according to claim 1 wherein the active reagent is cephalin and a binder solution containing an activator is mixed with the reagent solution prior to lyophilization.

22. The process according to claim 21 wherein the activator is a particulate activator.

23. The process according to claim 22 wherein the activator is fumed silica.

24. The process according to claim 23 wherein the fumed silica has a size no greater than 0.012 microns.

25. The process according to claim 21 wherein the cephalin is mixed with a saline solution containing a preservative prior to lyophilization.

26. The process according to claim 25 wherein the preservative is thimerosal.

27. The process according to claim 21 wherein the buffer is tris, and the binder solution is a malto-dextrin solution.

28. The process according to claim 1 wherein the active reagent is a plasma control selected from the group consisting of fresh normal plasma drawn in CPDA-1, an abnormal control having less than normal clotting factors, and heparinized plasma.

29. The process according to claim 28 wherein the binder is a combination of a sugar selected from the group consisting of trehalose and sucrose, and a mixture of about 97.0% sucrose and about 3.0% malto-dextrin.

30. The process according to claim 29 wherein the sugar is an amount of about 0.5% by weight to about 20.0% by weight and the mixture of sucrose and malto-dextrin is in an amount of about 5.0% by weight to about 50.0% by weight.

31. The process according to claim 29 wherein the sugar is added to the active reagent prior to lyophilization and the mixture of sucrose and malto-dextrin is dry blended with the lyophilized active reagent.

32. The process according to claim 28 wherein the lubricant is dry blended with the lyophilized active reagent in an amount of about 1.0% to about 15.0% by weight.

33. The process according to claim 28 wherein the lubricant is selected from the group consisting of L-leucine, polyethylene glycol, and glycine.

34. The process according to claim 28 wherein the lubricant is no more than about 1% by weight of magnesium stearate.

35. The process according to claim 28 wherein up to about 5.0% by weight of a disintegrating agent is dry blended with the lyophilized active reagent.

36. The process according to claim 28 wherein a sugar selected from the group consisting of trehalose and sucrose in an amount of about 0.5% to 20.0% by weight is added to the active reagent prior to lyophilization, wherein a binder of about 97.0% sucrose and about 3.0% malto-dextrin is dry blended with the lyophilized active reagent in an amount from about 5.0% to about 50.0% by weight, and wherein the lubricant is polyethylene glycol which is dry blended with the lyophilized active reagent in an amount of about 1.0% to about 15.0% by weight.

37. The process according to claim 36 wherein up to about 5% by weight of a disintegrating agent is dry blended with the lyophilized active reagent.

38. The process according to claim 1 wherein the binder is added to the active reagent solution prior to lyophilization; the buffer is lyophilized, compacted and granulated; and the active reagent/binder solution is sprayed on the granulated buffer prior to the compacting step.

39. The process according to claim 38 wherein the lubricant is added after the granulating step and prior to the tableting step.

40. The process according to claim 38 wherein the active reagent is thromboplastin phospholipid which is dissolved in a calcium chloride solution, the binder is trehalose in an amount of about 1.0% to about 10.0% by weight, a divalent metal selected from the group consisting of magnesium and manganese is added to the buffer prior to lyophilization, the buffer is imidazole having a pH of about 7.35, and the lubricant is polyethylene glycol in an amount of about 1.0% to about 12.0% by weight.

41. A microconcentrated blood plasma coagulation reagent tablet comprising an active reagent selected from the group consisting of thrombin, thromboplastin, cephalin, fresh normal plasma drawn in CPDA-1, abnormal plasma having less than normal clotting factors, and heparinized plasma; a buffer selected from the group consisting of HEPES, imidazole, tris, glycine, and barbitol; a binder; and a lubricant.

42. The tablet according to claim 41 wherein the active reagent is lyophilized thrombin, the binder is in an amount from about 20.0% to about 90.0% by weight, and the lubricant is in an amount from about 1.0% to about 12.0% by weight.

43. The tablet according to claim 42 wherein the binder is selected from the group consisting of about 97.0% sucrose and about 3.0% malto-dextrin, sucrose, maltose, mannitol and lactose.

44.   The tablet according to claim 42 wherein the lubricant is selected from the group consisting of L-leucine, polyethylene glycol, magnesium stearate and talc.

45.   The tablet according to claim 42 including sodium chloride, a preservative selected from the group consisting of thimerosal and sodium azide, and bovine serum albumin.

46.   The tablet according to claim 41 wherein the active reagent is lyophilized thromboplastin and the tablet includes calcium chloride.

47.   The tablet according to claim 46 wherein the buffer is selected from the group consisting of imidazole, tris, and barbitol.

48.   The tablet according to claim 46 including a divalent metal other than calcium.

49.   The tablet according to claim 48 wherein the divalent metal is selected from the group consisting of magnesium and manganese.

50.   The tablet according to claim 46 including a preservative selected from the group consisting of thimerosal, neomycin sulfate, sodium azide, and phenol.

51.   The tablet according to claim 46 wherein the binder is malto-dextrin in an amount from about 1% to about 10.0% by weight.

52.   The tablet according to claim 46 wherein the lubricant is polyethylene glycol in an amount from about 1.0% to about 12.0%.

53.   The tablet according to claim 46 wherein the lubricant is selected from the group consisting of polyethylene glycol, magnesium stearate, L-leucine, glycine and silica.

54.   The tablet according to claim 41 wherein the active reagent is cephalin and the binder contains an activator.

55.   The tablet according to claim 54 wherein the activator is fumed silica.

56. The tablet according to claim 54 wherein the activator is a particulate activator.

57. The tablet according to claim 54 wherein the fumed silica is no greater than 0.012 microns in size.

58. The tablet according to claim 54 wherein the buffer is tris, the binder is malto-dextrin containing fumed silica, and the tablet contains saline and thimerosal.

59. The tablet according to claim 41 wherein the active reagent is selected from the group consisting of fresh normal plasma drawn in CPDA-1, abnormal plasma having less than normal clotting factors and a heparinized plasma, wherein the binder is a sugar in an amount from about 5.0% to about 50.0% by weight, and wherein the lubricant is in an amount of about 1.0% to about 15.0% by weight.

60. The tablet according to claim 59 wherein the binder is a combination of a sugar selected from the group consisting of sucrose and trehalose and about 97.0% sucrose and about 3.0% malto-dextrin.

61. The tablet according to claim 59 wherein the lubricant is selected from the group consisting of L-leucine, polyethylene glycol, and glycine.

62. The tablet according to claim 59 wherein the tablet includes a disintegrating agent in an amount up to about 5%.

63. The tablet according to claim 62 wherein the disintegrating agent is selected from the group consisting of cellulose, starch and effervescent mixtures.

64. The tablet according to claim 59 wherein the lubricant is polyethylene glycol.